(19) Europäisches Patentamt
European Patent Office
Office européen des brevets

(11) Publication number: **0 250 358 A2**

# (12) EUROPEAN PATENT APPLICATION

(21) Application number: 87730065.7

(22) Date of filing: 15.06.87

(51) Int. Cl.[4]: **C 07 D 211/26**
C 07 D 207/09,
C 07 D 211/56,
C 07 D 207/14, C 07 F 9/56,
A 61 K 49/00
// C07D413/14

(30) Priority: 20.06.86 DE 3621025
20.06.86 DE 3621026

(43) Date of publication of application:
23.12.87 Bulletin 87/52

(84) Designated Contracting States:
AT BE CH DE ES FR GB GR IT LI LU NL SE

(71) Applicant: SCHERING AKTIENGESELLSCHAFT Berlin und Bergkamen
Müllerstrasse 170/178 Postfach 65 03 11
D-1000 Berlin 65 (DE)

(72) Inventor: Gries, Heinz, Dr.
Helmstedter Strasse 19
D-1000 Berlin 31 (DE)

Renneke, Franz-Josef, Dr.
Hochstrasse 67
D-4709 Bergkamen (DE)

Weinmann, Hans-Joachim, Dr.
Ahornstrasse 31
D-1000 Berlin 41 (DE)

## (54) Novel complex compounds.

(57) Complex compounds of general Formula I

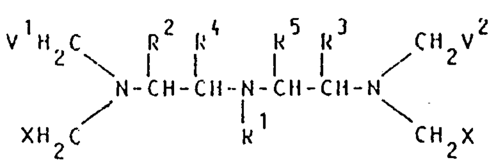

(I)

wherein
X means the residues -COOY and $PO_3HY$ with Y meaning a hydrogen atom, a metal ion equivalent and/or a physiologically acceptable cation of an inorganic or organic base or amino acid,
$V^1$ is the residue X, a

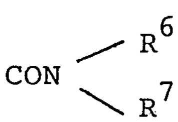

or $COOR^{12}$ group

wherein
$R^6$ stands for a saturated, unsaturated, straight- or branched-chain or cyclic hydrocarbon residue of up to l6 carbon atoms optionally substituted by l-5 hydroxy groups or an aryl or aralkyl group optionally substituted by one or several $C_1$-$C_6$-dialkylamino or one or several $C_1$-$C_6$-alkoxy groups,
$R^7$ stands for a hydrogen atom, for a saturated, unsaturated, straight- or branched-chain or cyclic hydrocarbon residue of up to l6 carbon atoms optionally substituted by l-5 hydroxy groups,
or $R^6$ and $R^7$ jointly stand for a saturated or unsaturated 5- or 6-membered ring optionally substituted by one or several $C_1$-$C_5$-alkyl, $C_1$-$C_5$-hydroxyalkyl, an optionally hydroxylated or $C_1$-$C_5$-alkoxylated $C_2$-$C_6$-acyl, hydroxy, carbamoyl, carbamoyl-substituted $C_1$-$C_6$-alkyl, carbamoyl substituted at nitrogen by one or two $C_1$-$C_6$-alkyl residue(s) -- which also can form a ring optionally containing an oxygen atom -- or a $C_1$-$C_5$-acylamino or alkylacylamino residue, this 5- or 6-membered ring optionally containing a further nitrogen, oxygen, sulfur atom, or a carbonyl group, and
$R^{12}$ stands for a saturated, unsaturated, straight- or branched-chain or cyclic hydrocarbon residue of up to l6 carbon atoms, or an aryl or aralkyl group,
$V^2$ means the residue X,
a

EP 0 250 358 A2

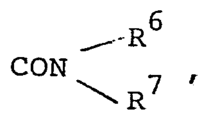

$COOR^{12}$ or COB group wherein B is a macromolecule residue,
$R^1$ is a hydrogen atom or a $CH_2X$ group,
$R^2$ and $R^3$ mean a $(CH_2)_m$ group with m meaning 0 or I if $R^4$ and $R^5$ are simultaneously a hydrogen atom each,
$R^4$ and $R^5$ are a

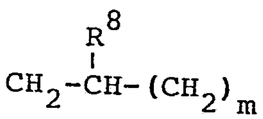

group with m meaning 0 or I, $R^8$ meaning a hydrogen atom or $R^9$,
wherein $R^9$ stands for a straight-chain or branched, saturated or unsaturated $C_1$-$C_{20}$-alkylene group which exhibits at the end either a functional group or, bound via the latter, a macromolecule B and contains optionally imino, phenylenoxy, phenylenimino, amide, ester group(s), oxygen, sulfur and/or nitrogen atom(s) and is optionally substituted by hydroxy, mercapto, imino and/or amino group(s), if simultaneously $R^2$ and $R^3$ each stand for a hydrogen atom,
with the proviso that, if $V^2$ stands for a COB group, $R^8$ means a hydrogen atom and that, if X stands for the group $PO_3HY$, $V^1$ and $V^2$ likewise mean $PO_3HY$,

are valuable complex compounds.

**Description**

NOVEL COMPLEX COMPOUNDS

The invention relates to the subject matter characterized in the claims, i.e. novel complexing agents, complexes, and complex salts, agents containing these compounds, their use in diagnostics and therapy, as well as processes for the preparation of these compounds and agents.

Complexes and, respectively, the salts thereof have been utilized in medicine for some time, thus, for example, as auxiliary agents to administer poorly soluble ions (e.g. iron) and as antidotes (in this connection, calcium or zinc complexes are preferred) for the detoxification in case of inadvertent incorporation of heavy metals or their radioactive isotopes.

In Patents EP 7l564, EP l30934 and DOS 340l052, complexes and complex salts have recently been disclosed as diagnostic agents, predominantly as NMR diagnostic agents.

All of the heretofore known complexes and their salts cause problems in their clinical application with respect to the compatibility and/or selectivity of binding and/or stability. These problems become the more pronounced, the higher the dosages that must be used for the products derived from the complexing agents. For example, use is limited in the therapy of metal poisonings on account of the inadequate renal compatibility of the compounds available at present, as well as their tendency to bind ions essential to the organism. Thus far, the actually beneficial utilization of heavy elements as components of X-ray contrast media to be administered parenterally failed on account of inadequate compatibility of such compounds. In the paramagnetic, contrast-enhancing substances heretofore proposed or tested for nuclear spin tomography, the gap between the effective dose and the dose that is toxic in animal experiments is relatively narrow and/or the substances exhibit a low organ specificity and/or stability and/or contrast-enhancing effect.

Consequently, for a variety of purposes, there is a need for complex compounds which, above all, show improved compatibility, but also stability, good solubility and adequate selectivity.

It has now been discovered that complex compounds consisting of the anion of a complex-forming acid and a central ion of an element of atomic numbers 2l-29, 3l, 32, 38, 39, 42-44, 49, 57-70 or 77 and optionally one or several physiologically acceptable cations of an inorganic and/or organic base or amino acid surprisingly are excellently suitable for the preparation of pharmaceutical media suited for use in the NMR, X-ray, ultrasonic, radiodiagnostics or radiotherapy.

They are described by general Formula I

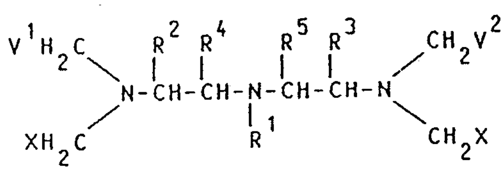

(I)

wherein

X means the residues -COOY and $PO_3HY$ with Y meaning a hydrogen atom, a metal ion equivalent and/or a physiologically acceptable cation of an inorganic or organic base or amino acid,

$V^1$ is the residue X, a

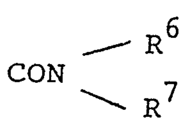

or $COOR^{12}$ group wherein

$R^6$ stands for a saturated, unsaturated, straight- or branched-chain or cyclic hydrocarbon residue of up to l6 carbon atoms optionally substituted by l-5 hydroxy groups or an aryl or aralkyl group optionally substituted by one or several Cl-C6-dialkylamino or one or several $C_1$-$C_6$-alkoxy groups,

$R^7$ stands for a hydrogen atom, for a saturated, unsaturated, straight- or branched-chain or cyclic hydrocarbon residue of up to l6 carbon atoms optionally substituted by l-5 hydroxy groups, or $R^6$ and $R^7$ jointly stand for a saturated or unsaturated 5- or 6-membered ring optionally substituted by one or several $C_1$-$C_5$-alkyl, $C_1$-$C_5$ hydroxyalkyl, an optionally hydroxylated or $C_1$-$C_5$-alkoxylated $C_2$-$C_6$-acyl, hydroxy, carbamoyl, carbamoyl-substituted $C_1$-$C_6$-alkyl, carbamoyl substituted at nitrogen by one or two $C_1$-$C_6$-alkyl residue(s) -- which also can form a ring optionally containing an oxygen atom -- or a $C_1$-$C_5$-acylamino or alkylacylamino residue, this 5- or 6-membered ring optionally containing a further nitrogen, oxygen, sulfur atom, or a carbonyl group, and $R^{12}$ stands for a saturated, unsaturated, straight- or branched-chain or cyclic hydrocarbon residue of up to l6 carbon atoms, or an aryl or aralkyl group,

$V^2$ means the residue X,

a

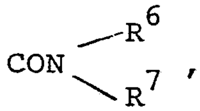

$COOR^{12}$ or COB group wherein B is a macromolecule residue,
$R^1$ is a hydrogen atom or a $CH_2X$ group,
$R^2$ and $R^3$ mean a $(CH_2)_m$ group with m meaning 0 or l if $R^4$ and $R^5$ are simultaneously a hydrogen atom each,
$R^4$ and $R^5$ are a

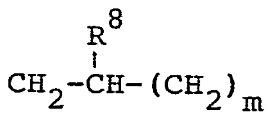

group with m meaning 0 or l, $R^8$ meaning a hydrogen atom or $R^9$,
wherein $R^9$ stands for a straight-chain or branched, saturated or unsaturated $C_1$-$C_{20}$-alkylene group which exhibits at the end either a functional group or, bound via the latter, a macromolecule B and contains optionally imino, phenylenoxy, phenylenimino, amide, ester group(s), oxygen, sulfur and/or nitrogen atom(s) and is optionally substituted by hydroxy, mercapto, imino and/or amino group(s), if simultaneously $R^2$ and $R^3$ each stand for a hydrogen atom,
with the proviso that, if $V^2$ stands for a COB group, $R^8$ means a hydrogen atom and that, if X stands for the group $PO_3HY$, $V^1$ and $V^2$ likewise mean $PO_3HY$.

The element of the above-mentioned atomic number constituting the central ion of the physiologically compatible complex salt can, of course, also be radioactive for the desired purpose of using the diagnostic agent of this invention.

If the agent of this invention is intended for NMR diagnostics (see European Patent Application No. 7l564), then the central ion of the complex salt must be paramagnetic. This is true, in particular, for the divalent and trivalent ions of the elements of atomic numbers 2l-29, 42, 44 and 57-70. Suitable ions are, for example, the chromium(III), manganese(II), iron(III), iron(II), cobalt(II), nickel(II), copper(II), praseodymium(III), neodymium(III), samarium(III) and ytterbium(III) ions. Due to their strong magnetic moment, especially preferred are the gadolinium(III), terbium(III), dysprosium(III), holmium(III), erbium(III) and iron(III) ions.

If the agent of this invention is intended for use in X-ray diagnostics, then the central ion must be derived from an element having a higher atomic number to attain adequate absorption of the X-rays. It has been found that diagnostic agents are suitable for this purpose which contain a physiologically compatible complex salt with central ions of elements of atomic numbers of between 57 and 70; these are, for example, the lanthanum(III) ion and the above-mentioned ions of the lanthanide series.

The agents of this invention intended for use in NMR diagnostics, as well as those destined for utilization in X-ray diagnostics are suited for application in ultrasonic diagnostics.

For use of the agents of this invention in nuclear medical diagnostics and therapy, the central ion must be radioactive. Suitable are, for example, radioisotopes of the elements copper, cobalt, gallium, germanium, yttrium, strontium, indium, ytterbium, gadolinium, samarium, iridium and, in particular, technetium (Tc-99m).

Suitable alkyl substituents $R^6$, $R^7$ and $R^{12}$ are saturated, unsaturated, straight- or branched-chain or cyclic hydrocarbons of up to l6 carbon atoms, preferably saturated hydrocarbons of l-l0 carbon atoms, especially saturated hydrocarbons of l-5 carbon atoms which, in case of $R^6$ and $R^7$, are optionally substituted by l-5, preferably 2-4 hydroxy groups. Examples that can be cited are: methyl, ethyl, propyl, isopropyl, butyl, isobutyl, pentyl, cyclopentyl, cyclohexyl, propenyl.

Examples for suitable hydroxyalkyl residues are: 2-hydroxypropyl, 3-hydroxypropyl, l-(hydroxymethyl)ethyl, 2,3-dihydroxypropyl, tris(hydroxymethyl)methyl, 2,3-dihydroxy-l-hydroxymethylpropyl, 2,3,4,5,6-pentahydroxyhexyl and preferably 2-hydroxyethyl, 2-hydroxy-l-(hydroxymethyl)ethyl, 2,3-dihydroxypropyl and 2,3,4-trihydroxybutyl.

In case $R^7$ stands for a hydrogen atom, $R^3$ can also be an aryl or aralkyl group, for example a phenyl or benzyl group, optionally substituted by one or several $C_1$ to $C_6$ dialkylamino groups or by one or several $C_1$ to $C_6$ alkoxy groups.

The heterocyclic 5- or 6-membered ring, formed from $R^6$ and $R^7$ with inclusion of the amide nitrogen, can be saturated, unsaturated and/or substituted and can optionally contain a nitrogen, oxygen, sulfur atom or a carbonyl group.

The heterocycle can be substituted by a hydroxy group, a $C_1$-$C_6$-alkyl group, e.g. methyl, ethyl, propyl, isopropyl, butyl, a $C_1$-$C_5$-hydroxyalkyl group, e.g. hydroxymethyl, hydroxyethyl, or by a $C_2$-$C_6$-acyl group, e.g. acetyl, propionyl, which can be substituted, if desired, by a hydroxy or $C_1$-$C_6$-alkoxy group, for example, methoxy, ethoxy.

Another substituent that can be mentioned is the carbamoyl group which is bound to the heterocycle directly or separated by a $C_1$-$C_6$-alkylene group, for example methylene, ethylene, propylene, and is optionally substituted on the nitrogen by one or two $C_1$-$C_6$-alkyl residue(s), e.g. methyl, ethyl, propyl, isopropyl, optionally forming a ring, such as, for example, a pyrrolidine or piperidine ring. The carbamoyl nitrogen can also be part of a morpholine ring.

Another possible substituent at the heterocycle that can be cited is an optionally $C_1$-$C_6$-alkylated or $C_1$-$C_6$-acylated primary or secondary amino group, such as, for example, the methyl-, ethyl-, acetyl-, propionylamino groups.

Examples of suitable heterocycles are: the pyrrolidinyl, piperidyl, pyrazolidinyl, pyrrolinyl, pyrazolinyl, piperazinyl, morpholinyl, imidazolidinyl, oxazolidinyl, thiazolidinyl rings.

The alkylene group contained in $R^9$ can be straight-chain, branched, cyclic, aliphatic, aromatic or arylaliphatic and can have up to 20 carbon atoms. Preferred are straight-chain mono- to hexamethylene groups as well as $C_1$-$C_4$-alkylenephenyl groups. If the alkylene group contains a phenoxy group, then this group is preferably bound in the p-position via a methylene group to the -CH- group of the basic skeleton of the compound of general Formula I.

Preferred functional groups present at the end of the $R^9$ alkylene group are, for example, the benzyl ester, ethyl ester, tert-butyl ester, amino, $C_1$-$C_6$-alkylamino, aminocarbonyl, hydrazino, hydrazinocarbonyl, maleimido, methacrylamido, methacryloylhydrazinocarbonyl, maleimidamidocarbonyl, halogeno, mercapto, hydrazinotrimethylenehydrazinocarbonyl, aminodimethylenamidocarbonyl, bromocarbonyl, phenylenediazonium, isothiocyanate, semicarbazide, thiosemicarbazide groups.

For clarification, several selected $R^9$-substituents will be set forth below:

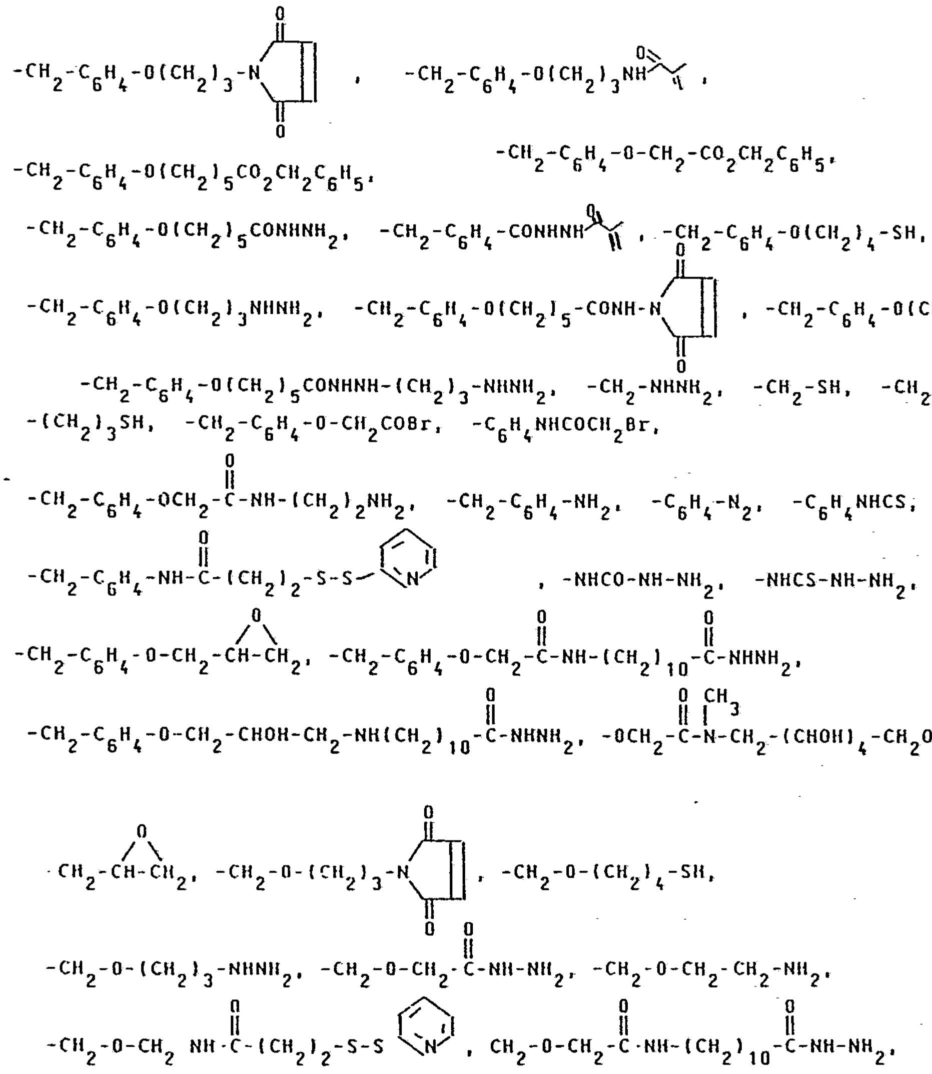

If not all of the acidic hydrogen atoms are substituted by the central ion, it is possible, if desired, to replace one, several ones, or all remaining hydrogen atom(s) by physiologically acceptable cations of inorganic and/or organic bases or amino acids. Suitable inorganic cations are, for example, the lithium ion, the potassium ion, the calcium ion and, in particular, the sodium ion. Suitable cations of organic bases are, inter alia, those of primary, secondary or tertiary amines, e.g. ethanolamine, diethanolamine, morpholine, glucamine, N,N-dimethylglucamine and, in particular, N-methylglucamine. Suitable cations of amino acids are, for example, those of lysine, arginine and ornithine.

The complexing acids can also be linked to macromolecules, of which it is known that they are especially enriched in the organ or organ part to be investigated. Such macromolecules are, for example, hormones, dextrans, polysaccharides, polychelones, hydroxyethyl starch, polyethylene glycol, desferrioxamines, bleomycins, insulin, prostaglandins, steroid hormones, amino sugars, amino acids, peptides, such as polylysine, proteins (such as, for example, immunoglobulins and monoclonal antibodies) or lipids (also in the form of liposomes). Conjugates with albumins, such as human serum albumin, with antibodies, such as, for example, monoclonal antibodies specific for tumor-associated antigens, or antimyosin deserve special mention. It is also possible to add by linkage suitable synthetic polymers, such as polyethylenimines. The

diagnostic media formed therefrom are suited, for example, for use in tumor and infarction diagnostics. Especially suitable monoclonal antibodies for conjugation are those directed against predominantly cell-membrane-fixed antigens. Suitable as such antibodies are, for example, monoclonal antibodies and/or their fragments [F(ab)2] for tumor imaging, directed, for example, against the carcinoembryonic antigen (CEA), human chorionic gonadotropin (β-hCG), or other tumor-fixed antigens, such as glucoproteins. Also suitable, inter alia, are anti-myosin, anti-insulin and anti-fibrin antibodies.

Suitable for liver tests and/or for tumor diagnostics are, for example, conjugates or clathrates with liposomes (used, for instance, as unilaminar or multilaminar phosphatidylcholine cholesterol vesicles).

Formation of the conjugate takes place either via a carboxyl group of the complex-forming acid or via the functional group, as defined above, located at the end of the $C_1$-$C_{20}$-alkylene group of substituent $R^9$. When forming the conjugate of the complex-forming acids with macromolecules, several acid residues can be bound thereto. In this case, each complex-forming acid residue can carry a central ion.

Coupling to the desired macromolecules likewise takes place according to known methods as described, for example, in Rev. roum. Morphol. Embryol. Physiol., Physiologie l98l, l8 : 24l, and J. Pharm. Sci. 68 : 79 (l979), for example by reacting the nucleophilic group of a macromolecule, such as the amino, phenol, sulfhydryl, aldehyde or imidazole group with an activated derivative of the complexing agent. Examples for activated derivatives are monoanhydrides, acid chlorides, acid hydrazides, mixed anhydrides (see, for example, G.E. Krejcarek and K.L. Tucker, Biochem. Biophys. Res. Commun. 30 l977, 58l), activated esters, nitrenes or isothiocyanates. Conversely, it is also possible to react an activated macromolecule with the complexing acid. Also suitable for conjugation with proteins are substituents, for example, of the structure $C_6H_4N_2$, $C_6H_4NHCOCH_2$, $C_6H_4NHCS$ or $C_6H_4OCH_2CO$.

The complex compounds of general Formula I according to this invention are prepared in that, in a manner known per se, amines of general Formula II

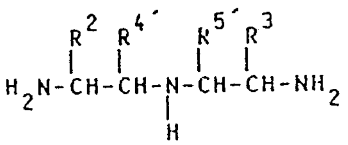

(II),

wherein

$R^2$ and $R^3$ represent a $(CH_2)_m$ group where m means 0 or l if simultaneously $R^{4'}$ and $R^{5'}$ each stand for a hydrogen atom,

$R^{4'}$ and $R^{5'}$ represent a

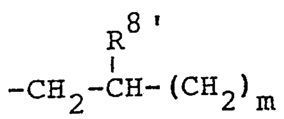

group wherein $R^{8'}$ is a hydrogen atom or $R^{9'}$, wherein $R^{9'}$ stands for a straight-chain, branched, saturated or unsaturated $C_1$-$C_{20}$-alkylene group exhibiting at the end a group that can be converted into the functional group contained in $R^9$ and optionally containing imino, phenylenoxy, phenylenimino, amide ester group(s), oxygen, sulfur and/or nitrogen atom(s) and being optionally substituted by hydroxy, mercapto, imino and/or amino group(s),

are alkylated with

(a) phosphorous acid/formaldehyde or in the presence of a base

(b) a compound $HalCH_2COOR^{10}$ wherein Hal is chlorine, bromine or iodine and $R^{10}$ is a hydrogen atom or an alkyl residue of l-4 carbon atoms,

and subsequently alkyl residues $R^{10}$ that may be present are split off by saponification and, if desired, the thus-obtained acetic-acid-substituted compounds are reacted, by way of compound III, IV or V

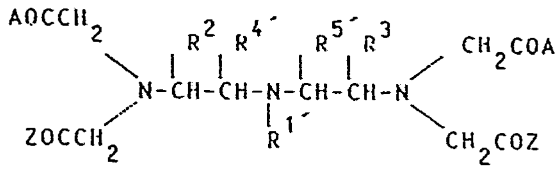

(III),

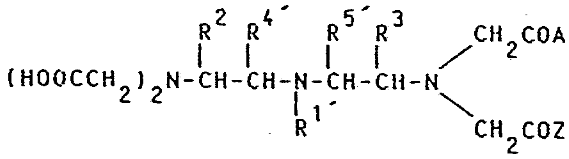

(IV),

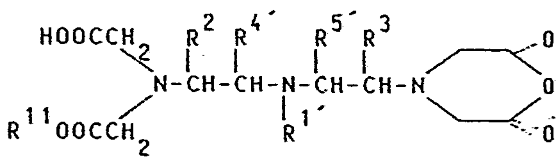

(V),

wherein
$R^{1'}$ is a hydrogen atom or a $CH_2COOH$ group,
$R^{11}$ is a $C_1$-$C_6$-alkyl residue,
A and Z jointly represent an oxygen atom, or A is a hydroxy group and Z is the grouping $OR^{11}$, with an amine of general Formula VI

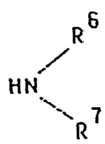

(VI),

and optionally still present ester groups are saponified or, respectively, bisanhydrides of general Formula IIIa

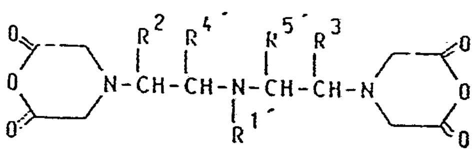

(IIIa)

are reacted with an alcohol $R^{12}OH$,
the thus-obtained acids of general Formula I wherein Y means a hydrogen atom, if desired,

(a) are conventionally reacted with at least one metal oxide or metal salt of an element of atomic numbers 21-29, 31, 32, 38, 39, 42-44, 49 or 57-70 or 77 and subsequently, if desired, acidic hydrogen atoms present are substituted by cations of inorganic and/or organic bases or amino acids,
or

(b) are conventionally reacted with at least one metal oxide or metal salt of an element of atomic numbers 21-29, 31, 32, 38, 39, 42-44, 49, 57-70 or 77 and subsequently the thus-obtained metal complexes are conventionally bound to a macromolecule by way of the functional group contained in $R^{9'}$ and, respectively, to the CO group contained in $V^2$, and, if desired, acidic hydrogen atoms present are substituted by cations of inorganic and/or organic bases or amino acids,
or

(c) are bound conventionally to a macromolecule by way of the functional group contained in $R^{9'}$ and, respectively, to the CO group contained in $V^2$, and subsequently, in a manner known per se, are reacted with at least one metal oxide or metal salt of an element of atomic numbers 21-29, 31, 32, 38, 39, 42-44, 49, 57-70 or 77 and thereafter, if desired, acidic hydrogen atoms present are substituted by cations of inorganic and/or organic bases or amino acids.

The optionally substituted 3,4-diamino-, 2,5-bis(aminomethyl)pyrrolidine, 3,5-diamino- and 2,6-bis(aminomethyl)piperidine serving as the educts are amines of general Formula II

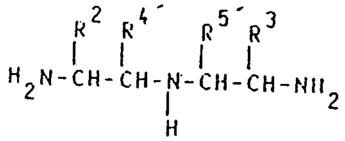

(II),

wherein $R^2$, $R^3$, $R^{4'}$ and $R^{5'}$ have the meanings given above. They are known from the literature (J. Pharm. Sci. 58 : l038, l969) and, respectively, are obtained in a manner known per se by catalytic hydrogenations, for example with rhodium/carbon or Nishimura catalyst as the catalyst (Houben-Weyl, Methoden der organischen Chemie [Methods of Organic Chemistry] 4/lc : 256, Georg Thieme Publishers, Stuttgart, New York l980, F. Zymalkowsky, Katalytische Hydrierungen [Catalytic Hydrogenations], Ferdinand Enke Publishers Stuttgart l965) from corresponding aromatic precursors (Rec. de Travau de Chim. des Pays-Bas 72 : 569 [l953], Ann. Chem. 537 [l978], J. Pharm. Soc. Japan 79 : 549 [l959]).

Amines with the respectively desired $R^{9'}$ substituent are obtained analogously by starting with suitably substituted educts (e.g. chelidamic acid) and then introducing the substituent according to methods known from the literature (e.g. alkylation with bromobenzyl derivatives).

Suitable substituents $R^{9'}$ which can be converted into the substituents $R^9$ exhibiting at the end a functional group suited for linkage to a macromolecule are, inter alia, hydroxy- and nitrobenzyl, hydroxy- and carboxyalkyl, as well as thioalkyl residues of up to l0 carbon atoms. They are converted, according to methods in the literature known to persons skilled in the art (Chem. Pharm. Bull. 33 : 674 [l985], Compendium of Org. Synthesis l-5, Wiley and Sons, Inc.), into the desired substituents (for example with the amino, hydrazino, hydrazinocarbonyl, methacryloylhydrazinocarbonyl, maleimidamidocarbonyl, halogeno, halogenocarbonyl, mercapto group as the functional group) wherein, in case of the nitrobenzyl residue, first a catalytic hydrogenation (e.g. according to P.N. Rylander, Catalytic Hydrogenation over Platinum Metals, Academic Press l967) must be performed to obtain the aminobenzyl derivative. Examples for the conversion of hydroxy or amino groups are the reactions with a substrate of general Formula VII

W-L-Fu (VII)

wherein W is a nucleofugic entity such as, for example, Cl, Br, I, $CH_3C_6H_4SO_3$ or $CF_3SO_3$; L is an aliphatic, aromatic, arylaliphatic, branched, straight-chain or cyclic hydrocarbon residue of up to 20 carbon atoms; and Fu is the desired end-positioned functional group,
which reactions are performed in anhydrous, aprotic solvents such as tetrahydrofuran, dimethoxyethane or dimethyl sulfoxide in the presence of an acid captor such as, e.g. sodium hydroxide, sodium hydride or alkali or alkaline earth carbonates, e.g. sodium, magnesium, potassium, calcium carbonate, at temperatures of between 0° C and the boiling point of the respective solvent, but preferably between 20° C and 60° C.

Examples of compounds of general Formula VII are:

$Br(CH_2)_2NH_2$, $Br(CH_2)_3OH$, $BrCH_2COOCH_3$, $BrCH_2CO_2{}^tBu$,
$Br(CH_2)_4CO_2C_2H_5$, $BrCH_2COBr$, $BrCH_2CONH_2$, $ClCH_2COOC_2H_5$,
$BrCH_2CONHNH_2$, 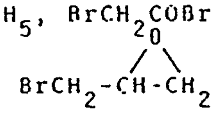, $CF_3SO_3(CH_2)_3Br$, $BrCH_2C{\equiv}CH$, $BrCH_2CH{=}CH_2$.

Conversions of carboxy groups can be conducted, for example, according to the carbodiimide method (Fieser, Reagents for Organic Syntheses l0 : l42) by way of a mixed anhydride (Org. Prep. Proc. Int. 7 : 2l5 [l975]) or by way of an activated ester (Adv. Org. Chem. Part B : 472).

For the synthesis of the compounds of general Formula I according to this invention, carrying phosphonic acid groups, the amines of general Formula II are reacted according to methods known to one skilled in the art (Phosphorus and Sulfur, l983, l6 : 233) with formaldehyde/phosphorous acid.

By alkylation of the amines of general Formula II with haloacetic acids and, respectively, their esters, of the formula $HalCH_2COOR^{10}$ wherein Hal stands for chlorine, bromine or iodine and $R^{10}$ stands for a hydrogen atom or an alkyl residue of l-4 carbon atoms, it is possible to obtain, by choosing appropriate auxiliary bases, either the corresponding tetracarboxylic acids ($R^{1'}$ = H) or the esters thereof, or the corresponding pentacarboxylic acids ($R^{1'}$ = $CH_2COOH$) or the esters thereof.

Suitable auxiliary bases are all bases known to a person skilled in the art for alkylating reactions, such as, for example, alkali and alkaline earth carbonates, alkali and alkaline earth hydrogen carbonates, polymeric anion exchangers, as well as the usual organic auxiliary bases, such as, for example, triethylamine, tetramethylpiperidine, pentamethylpiperidine, protone sponge , and tetramethylguanidine. Lithium carbonate, sodium carbonate, and "Amberlite" LA2 are preferred for producing the tetraacetic-acid-substituted compounds; potassium carbonate, tetra- and pentamethylpiperidine are preferred for obtaining the compounds carrying pentaacetic acid residues.

Suitable solvents are all solvents usable for alkylations, preferably tetrahydrofuran, dimethylformamide, dimethylacetamide and dimethyl sulfoxide.

Tetraacid-substituted complexing agents (general Formula I wherein $R^1$ and Y mean a hydrogen atom) can also be prepared by catalytic hydrogenation of the corresponding aromatic tetracarboxylic acids and, respectively, the esters thereof according to the above-mentioned methods (Chem. Berichte 117 : 948 [1984]). Subsequent N-alkylation in a manner known per se yields the desired pentaacetic-acid-substituted complexing agents.

The required saponification of the acetic acid ester groups that may have been formed is conducted according to methods known to those skilled in the art, for example with alkaline catalysts, such as alkali or alkaline earth carbonates or hydroxides.

Introduction of amide groups for the production of compounds of general Formula I wherein $V^1$ and/or $V^2$ is a $CONR^6R^7$ group takes place by partial conversion of carboxy groups of the aminotetra- and aminopentacarboxylic acids, obtained in the manner described above, into amide and respectively mono- or polyhydroxyalkyl amide groups. All of the possibilities for synthesis known to one skilled in the art can be utilized for this process. One example therefor is the reaction of the anhydrides or esters of general Formulae III through V

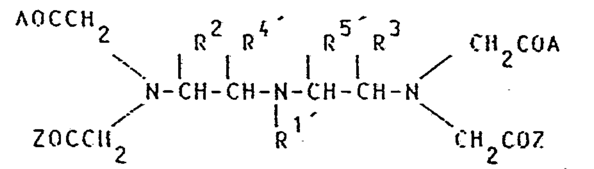

(III),

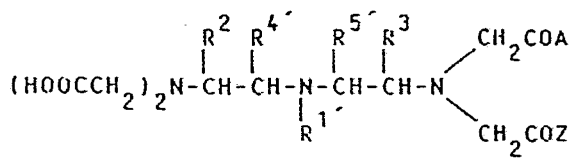

(IV),

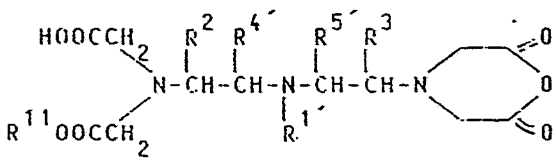

(V),

wherein

$R^{11}$ stands for a $C_1$-$C_6$-alkyl residue,

A and Z jointly mean an oxygen atom, or A means a hydroxy group and Z means the grouping $OR^{11}$, with amines of general Formula VI

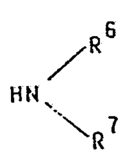

(VI)

wherein $R^6$ and $R^7$ have the meanings given above.

Suitable amines are, for example: dimethylamine, diethylamine, di-n-propylamine, diisopropylamine, di-n-butylamine, diisobutylamine, di-sec-butylamine, N-methyl-n-propylamine, dioctylamine, dicyclohexylamine, N-ethylcyclohexylamine, diisopropenylamine, benzylamine, aniline, 4-methoxyaniline, 4-dimethylaminoaniline, 3,5-dimethoxyaniline, morpholine, pyrrolidine, piperidine, N-methylpiperazine, N-ethylpiperazine, N-(2-hydroxyethyl)piperazine, N-(hydroxymethyl)piperazine, piperazinoacetic acid isopropylamide, N-(piperazinomethylcarbonyl)morpholine, N-(piperazinomethylcarbonyl)pyrrolidine, 2-(2-hydroxymethyl)piperidine, 4-(2-hydroxyethyl)piperidine, 2-hydroxymethylpiperidine, 4-hydroxymethylpiperidine, 2-hydroxymethylpyrrolidine, 3-hydroxypiperidine, 4-hydroxypiperidine, 3-hydroxypyrrolidine, 4-piperidone, 3-pyrroline, piperidine-3-carboxylic acid amide, piperidine-4-carboxylic acid amide, piperidine-3-carboxylic acid diethylamide, piperidine-4-carboxylic acid dimethylamide, 2,6-dimethylpiperidine, 2,6-dimethylmorpholine, N-acetylpiperazine, N-(2-hydroxypropionyl)piperazine, N-(3-hydroxypropionyl)piperazine, N-(methoxyacetyl)piperazine, 4-(N-acetyl-N-methylamino)piperidine, piperidine-4-carboxylic acid (3-oxapentamethylen)amide, piperidine-3-carboxylic acid (3-oxapenta methylen)amide, N-(N',N'-dimethylcarbamoyl)piperazine, pyrazoline, pyrazolidine, imidazoline, oxazolidine, thiazolidine, 2,3-dihydroxypropylamine, N-methyl-2,3-dihydroxypropylamine, 2-hydroxy-l-(hydroxymethyl)ethylamine, N,N-bis(2-hydroxyethyl)amine, N-methyl-2,3,4,5,6-pentahydroxyhexylamine, 6-amino-2,2-dimethyl-l,3-dioxepin-5-ol, 2-hydroxyethylamine, 2-amino-l,3-propanediol, diethanolamine, ethanolamine.

The polyhydroxyalkylamines can advantageously be used also in the blocked form for the reaction, for example as O-acyl derivatives or as ketals. This holds true especially if these derivatives can be manufactured more conveniently and more cheaply than the polyhydroxyalkylamines proper. A typical example is 2-amino-l-(2,2-dimethyl-l,3-dioxolan-4-yl)ethanol, the acetonide of l-amino-2,3,4-trihydroxybutane, prepared according to DOS 3l 50 9l7.

Subsequent removal of the blocking groups takes place without any problems and can be accomplished, for example, by treatment with an acidic ion exchanger in aqueous-ethanolic solution.

The acid anhydrides of general Formula III can be produced according to known methods, for example by following the mode of operation described in U.S. Patent 3,660,388 and, respectively, in DOS l6 95 050, with acetic anhdyride in pyridine.

However, in certain cases it is especially advantageous to effect the step of splitting off water in a gentle way with carbodiimides in a suitable solvent, such as, for example, dimethylformamide or dimethylacetamide.

The monoanhydrides of Formula IV can be prepared, for example, according to the method disclosed in J.A.O.C.S. 59 (2) : l05 (l982), see C.A. 96 : l64556 u (l982) by partial hydrolysis of bisanhydrides.

The preparation of the monoanhydrides of general Formula V shall be described, using as an example the monoanhydride of diethylenetriaminepentaacetic acid ethyl ester, starting with the monoethyl ester of DTPA (J. Pharm. Sci. 68 : l94, l979):

$N^3$-(2,6-Dioxomorpholinoethyl)-$N^6$-(ethoxycarbonylmethyl)-3,6-diazaoctanedioic Acid

A suspension of 2l.l g (50 mmol) of $N^3$,$N^6$-bis(carboxymethyl)-$N^9$-(ethoxycarbonylmethyl)-3,6,9-triazaundecanedioic acid in 250 ml of acetic anhydride is stirred at room temperature for three days after adding 42.2 ml of pyridine. The precipitate is then suctioned off, washed three times with respectively 50 ml of acetic anhydride, and stirred together with absolute diethyl ether subsequently for several hours. After suction removal, washing with absolute diethyl ether, and drying under vacuum at 40° C, l8.0 g (= 89% of theory) of a white powder is obtained having a melting point of l95-l96° C.

Analysis (based on anhydrous matter):

C 47.64 H 6.25 N l0.42 (calculated)

C 47.54 H 6.30 N l0.22 (found)

Reaction of the acid anhydrides to the amides of this invention is performed in the liquid phase. Suitable reaction media are, for example, water, dipolar aprotic solvents, such as acetonitrile, N-methylpyrrolidone, dimethylformamide, dimethylacetamide, and the like, or mixtures thereof. The reaction temperatures range between about 0° C and up to l00° C wherein temperatures of about 20° C to 80° C are preferred. The reaction periods range between 0.5 hour and 2 days, preferably between l hour and 36 hours.

The esters of general Formulae III and IV are produced conventionally, for example according to the processes disclosed in R.A. Guilmette et al. J. Pharm. Sci. 68 : l94 (l979), and IV in accordance with the process described in U.S. Patent 3,497,535.

The aminolysis of the esters takes place in the liquid phase, for example in a suitable, higher-boiling solvent, such as dimethylformamide, dimethylacetamide, or dimethyl sulfoxide. The reaction temperatures are about 20° C to 200° C, temperatures of l00-l80° C being preferred. The reaction times range between 2 hours and 2 days, reaction periods of between 4 and 36 hours being preferred.

The introduction of ester groups to prepare compounds of general Formula I wherein $V^1$ and/or $V^2$ stand for a $COOR^{12}$ group takes place according to methods known from the literature, for example by reacting bisanhydrides of general Formula IIIa

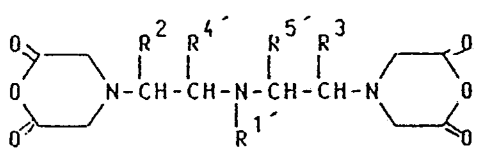

(IIIa)

with alcohols of the general formula $R^{12}OH$.

Moreover, all methods known to persons skilled in the art for converting carboxy groups into amide groups can be utilized for the synthesis of the complexing agents of this invention according to general Formula I, thus, for example, the method by Krejcarek and Tucker, Biochem. Biophys. Res. Commun. 77 : 58l (l977) by way of mixed anhydrides.

The metal complexes of this invention are prepared in the way described in patents EP 7l564, EP l30934 and DOS 34 0l 052 by dissolving or suspending the metal oxide or a metal salt (e.g. the nitrate, acetate, carbonate,

chloride or sulfate) of the element of atomic numbers 21-29, 31, 32, 38, 39, 42-44, 49, 57-70 or 77 in water and/or a lower alcohol (such as methanol, ethanol or isopropanol) and combining with a solution or suspension of the equivalent quantity of the complexing acid of general Formula I wherein Y means a hydrogen atom in water and/or a lower alcohol, and by stirring, if necessary while warming up or heating to the boiling point, until the reaction is finished. If the thus-formed complex salt is insoluble in the solvent employed, then it is isolated by filtration. If it is soluble, then it can be isolated by evaporation of the solution to dryness, for example by means of spray-drying.

If there are still acidic groups present in the resultant complex salt, it is frequently expedient to convert the acidic complex salt into neutral complex salts by means of inorganic and/or organic bases or amino acids which form physiologically acceptable cations, and to isolate these neutral complex salts. In many instances, this is even absolutely necessary since dissociation of the complex salt by shifting the pH value toward the neutral point is suppressed to such an extent as to even make it possible at all to isolate unitary products, or at least to purify such products.

Neutralization takes place herein with the aid of inorganic bases (e.g. hydroxides, carbonates or bicarbonates) of, for example, sodium, potassium or lithium and/or organic bases, such as, inter alia, primary, secondary and tertiary amines, such as, for example, ethanolamine, morpholine, glucamine, N-methyl- and N,N-dimethylglucamine, as well as basic amino acids, such as, for example, lysine, arginine and ornithine.

For producing the neutral complex compounds, such an amount of the desired bases can be added, for example, to the acidic complex salts in an aqueous solution or suspension that the neutral point is reached. The resultant solution can subsequently be concentrated to dryness under vacuum. It is frequently advantageous to precipitate the thus-formed neutral salts by adding water-miscible solvents, such as, for example, lower alcohols (methanol, ethanol, isopropanol, etc.), lower ketones (acetone etc.), polar ethers (tetrahydrofuran, dioxane, 1,2-dimethoxyethane, etc.), and thus to obtain crystallized products that can be readily isolated and easily purified. It proved to be especially advantageous to add the desired base as early as during the complex formation to the reaction mixture, thereby saving a process step.

If the acidic complex compounds contain several free acidic groups, it is often advantageous to produce neutral mixed salts containing inorganic as well as organic, physiologically acceptable cations as counter-ions. This can be done, for example, by reacting the complexing acid in an aqueous suspension or solution with the oxide or salt of the element yielding the central ion, and half the quantity of an organic base required for neutralization; isolating the thus-formed complex salt; optionally purifying same; and then combining same for complete neutralization with the required amount of inorganic base. The sequence of adding the bases can also be performed in the reverse order.

In case of using complex compounds that contain radioisotopes, these can be prepared according to the methods disclosed in "Radiotracers for Medical Applications" volume I, CRC Press, Boca Raton, Florida.

The diagnostic media of this invention are likewise prepared conventionally by suspending or dissolving the complex compounds of this invention, optionally with inclusion of the additives customary in galenic pharmacy, in an aqueous medium and then optionally sterilizing the suspension or solution. Suitable additives are, for example, physiologically acceptable buffers (such as, for example, tromethamine), small additions of complexing agents (such as, for example, diethylenetriaminepentaacetic acid) or, if necessary, electrolytes, such as, for example, sodium chloride or, if required, antioxidants, such as, for example, ascorbic acid.

If suspensions or solutions of the agents of this invention in water or physiological saline solution are desirable for enteral administration or other purposes, then they are mixed with one or several of the auxiliary agents customary in galenic pharmacy (for example, methylcellulose, lactose, mannitol) and/or tensides (for example lecithins, "Tweens", "Myrj") and/or flavoring materials to improve taste (e.g. ethereal oils).

In principle, it is also possible to prepare the diagnostic agents of this invention even without isolating the complex salts. In any event, special care must be directed toward effecting the chelate formation in such a way that the salts and salt solutions according to the invention are practically devoid of uncomplexed, toxically active metal ions.

This can be ensured, for example, with the aid of dye indicators, such as xylenol orange by control titrations during the manufacturing process. Therefore, the invention also relates to processes for the production of the complex compounds and their salts. Purification of the isolated complex salt remains as a final safety step.

If, for oral administration or other purposes, suspensions of the complex compounds in water or physiological saline solution are desired, then a poorly soluble complex compound is mixed with one or several galenically customary auxiliary agents and/or tensides and/or flavoring materials to improve taste.

The diagnostic media according to this invention preferably contain 1 μmol to 1 mol per liter of the complex salt and are normally made into dosages of 0.001 to 5 mmol/kg. They are intended for the enteral and parenteral administration.

The complex compounds of this invention are utilized

(1) for NMR, X-ray and ultrasonic diagnostics in the form of their complexes with the ions of the elements with atomic numbers 21-29, 42, 44 and 57-70;

(2) for radiodiagnostics and radiotherapy in the form of their complexes with the radioisotopes of the elements having atomic numbers 27, 29, 31, 32, 38,39, 43, 49, 64, 70 and 77.

The agents of this invention meet the variegated requirements for being suitable as contrast media for nuclear spin tomography. Thus, they are excellently suited for improving the informative content of the image obtained with the aid of the nuclear spin tomograph upon oral or parenteral administration, by increasing the

signal intensity. Furthermore, they exhibit the high efficacy necessary to introduce into the body a minimum amount of burdening foreign substances, and they show the good compatibility required for maintaining the noninvasive character of the examinations (for example, the compounds indicated in J. Comput. Tomography 5, 6:543-46 [I98I], in Radiology I44 : 343 [I982], and in Brevet Special de Medicament No. 484 M [I960] are too toxic).

The good water solubility of the agents of this invention makes it possible to prepare highly concentrated solutions, thus maintaining the volume load on the circulation within tolerable limits and compensating for dilution by body fluids.

Furthermore, the agents of this invention not only exhibit high stability in vitro, but also a high stability in vivo, so that a release or exchange of the ions that are toxic per se and are not bound in the complexes in a covalent fashion takes place only extremely gradually within the time period during which the novel contrast media are again completely eliminated. The conjugates with proteins and antibodies utilized, for example, in tumor diagnostics, effect a surprisingly high signal reinforcement already in such a low dose that it is here possible to utilize solutions having a correspondingly low concentration.

In general, the agents of this invention are dosed, for use as NMR diagnostics, in quantities of 0.00I to 5 mmol/kg, preferably 0.005 - 0.5 mmol/kg. Details of use are discussed, for example, in H.J. Weinmann et al., Am. J. of Roentgenology I42 : 6I9 (I984).

In contrast to the conventional X-ray diagnostics with radiopaque X-ray contrast media, there is, in NMR diagnostics with paramagnetic contrast media, no linear dependency of signal enhancement on the concentration employed. As has been revealed by control experiments, an increase in the dose applied does not absolutely result in signal amplification, and in case of a high dose of paramagnetic contrast media there may even occur extinction of the signal. For this reason, it was surprising that some pathological processes become visible only after administration of higher doses of a strongly paramagnetic contrast medium according to this invention. Thus, for example, confirmation of a defective blood-brain barrier in the zone of a cranial abscess can be obtained only upon administration of 0.05 - 2.5 mmol/kg, preferably 0.I - 0.5 mmol/kg, of paramagnetic complex salts such as, for example, gadolinium 2,6-bis[N,N-bis(carboxymethyl)aminomethyl]-I-piperidineacetic acid in the form of its well water-soluble salts. For a dose higher than 0.I mmol/kg, solutions are required having higher concentrations of up to I mol/l, preferably 0.25 - 0.75 mol/l since only in this way can the volume load be reduced and handling of the injection solution ensured.

Especially low dosages (below I mg/kg) of organ-specific NMR diagnostic agents are usable, for example, to detect tumors and cardiac infarctions. Furthermore, the complex compounds of this invention can be employed with advantage as shift reagents.

The agents of this invention are excellently suitable as X-ray contrast media; in this connection, it should be emphasized, in particular, that their use does not bring about any indications of anaphylaxis-type reactions in biochemical-pharmacological tests. They are especially valuable, on account of their favorable absorption properties in regions of higher tube voltages, for digital subtraction techniques.

In general, the agents of this invention are used, when administered as X-ray contrast media, in doses analogous to, for example, meglumine diatrizoate in amounts of 0.I - 5 mmol/kg, preferably 0.25 - I mmol/kg. Details of usage of X-ray contrast media are discussed, for example, in Barke, "Roentgenkontrastmittel" [X-Ray Contrast Media], G. Thieme, Leipzig I970, and P. Thurn, E. Bücheler, "Einfuehrung in die Roentgendiagnostik" [Introduction to X-Ray Diagnostics], G. Thieme, Stuttgart, New York I977.

The agents according to the invention are also suitable -- since their acoustic impedance is higher than that of body fluids and tissue -- as contrast media for ultrasonic diagnostics, especially in the form of suspensions. They are generally used in doses of amounts of 0.I - 5 mmol/kg, preferably 0.25 - I mmol/kg.

Details of the use of ultrasonic diagnostic media are disclosed, for example, in T.B. Tyler et al, Ultrasonic Imaging 3.323 (I98I), J.I. Haft, "Clinical Echokardiography", Futura, Mount Kisco, New York I978, and G. Stefan, "Echokardiographie" G. Thieme, Stuttgart/New York I98I.

The agents of this invention, due to their advantageous radioactive properties and good stability of the complex compounds contained therein, are likewise suitable as radiodiagnostic agents.

Details of their use and dosage are described, for example, in "Radiotracers for Medical Applications", CRC Press, Boca Raton, Florida.

The examples set forth below serve to provide a more detailed explanation of the present invention.

Example I

2,6-Bis[N,N-bis(carboxymethyl)aminomethyl]-I-piperidineacetic Acid

3I.6 g (55.0 mmol) of 2,6-bis[N,N-bis(ethoxycarbonylmethyl)aminomethyl]-I-piperidineacetic acid ethyl ester is dissolved in I60 ml of dioxane, and the solution is diluted with I60 ml of water. Under agitation and gradual heating to 80° C, 30.6 ml of IIN sodium hydroxide solution is added dropwise to this solution. The mixture is maintained at 80° C for I.5 hours and, after cooling to 20° C, adjusted to pH 2 with concentrated hydrochloric acid. The acidic solution is poured on a cation exchanger of the type "Amberlite" IR I20, the exchanger is washed with an ample amount of water, and then elution is carried out with semiconcentrated ammonia. The crude product obtained by evaporation of the eluate is again dissolved in 400 ml of water and adjusted to pH 2.6 by adding IR I20. The exchanger is suctioned off, and the aqueous solution is evaporated. The residue (I8.I g) is dissolved in I70 ml of methanol and I0 ml of water under heating and then gradually cooled, thus obtaining

13.8 g of colorless crystallized product (57% of theory), mp 189-191° C.
Analysis:
C 47.11 H 6.28 N 9.70 (calculated)
C 47.02 H 6.30 N 9.64 (found)
The starting material is prepared as follows:

(a) 2,6-Bis(aminomethyl)piperidine Trihydrochloride

123.3 g (0.5 mol) of 2,6-bis(aminomethyl)pyridine trihydrochloride (Annalen der Chemie 537-544 [1978]) is dissolved in 2.5 ml of water and, adding 25 g of rhodium/carbon as the catalyst, hydrogenated in an autoclave at maximally 50° C and under an initial pressure of 16 bar within one hour. The catalyst is filtered off and the colorless filtrate concentrated to dryness under vacuum. The oily residue is dissolved in 1,250 ml of methanol under heating and then again evaporated. The resultant foam is extracted by stirring in succession first in 600 ml of methanol and then in 600 ml of acetonitrile at 25° C, filtered, and dried under vacuum, yielding 109 g (86% of theory) of a colorless, strongly hygroscopic powder, mp 263-264° C.
Analysis:
C 33.28 H 7.98 N 16.64 Cl 42.10 (calculated)
C 33.31 H 7.91 N 16.50 Cl 42.13 (found)

(b) 2,6-Bis[N,N-bis(ethoxycarbonylmethyl)aminomethyl]-1-piperidineacetic Acid Ethyl Ester

Under agitation. 50.52 g (200 mmol) of 2,6-bis(aminomethyl)piperidine trihydrochloride is combined in 1,200 ml of dimethylacetamide with 165.8 g (1.2 mol) of potassium carbonate. After addition of 167 ml (1.5 mol) of bromoacetic acid ethyl ester, the mixture is heated for 24 hours to 100° C. After cooling to 25° C, the mixture is filtered and the filtrate concentrated under vacuum, thus obtaining as the residue a highly fluid oil which is taken up in 1,200 ml of ethyl acetate. The organic phase is washed four times with respectively 300 ml of water, dried over sodium sulfate, filtered, and the filtrate concentrated under vacuum, thus obtaining a crude product which is purified by way of column chromatography (material: silica gel) with methylene chloride/methanol (95:5) as the eluent, yielding 78 g (68% of theory) of a light-colored oil, the purity of which is determined by gas chromatography.
Analysis:
C 56.53 H 8.26 N 7.32 (calculated)
C 56.44 H 8.12 N 7.39 (found)

Example 2

2,6-Bis[N,N-bis(carboxymethyl)aminomethyl]piperidine

28.4 g (50 mmol) of 2,6-bis[N,N-bis(ethoxycarbonylmethyl)aminomethyl]-1-piperidine hydrobromide is dissolved in 200 ml of dioxane/water (1:1). Under stirring and heating to 80° C, 27 ml of 11N sodium hydroxide solution is added dropwise, and the solution is maintained at 80° C for 1.5 hours. After cooling to room temperature, the solution is acidified with concentrated hydrochloric acid to pH 2, and the acidic solution is poured on a cation exchanger of the type "Amberlite" IR 120. The exchanger is washed with an ample amount of water and then eluted with semi-concentrated ammonia. Evaporation of the eluate yields a crude product which is again dissolved in 300 ml of water. The solution is then adjusted to pH 3.0 with IR 120. The exchanger is suctioned off and the solution concentrated under vacuum. The residue is recrystallized from methanol/water (10:1), thus obtaining 13.5 g (72% of theory) of a colorless crystallized product, mp 129-132° C.
Analysis:
C 48.00 H 6.71 N 11.19 (calculated)
C 47.78 H 6.97 N 11.02 (found)
The starting material is produced as follows:

(a) 2,6-Bis[N,N-bis(ethoxycarbonylmethyl)aminomethyl]-1-piperidine Hydrobromide

A suspension is prepared from 25.2 g (100 mmol) of 2,6-bis(aminomethyl)piperidine trihydrochloride (produced according to Example 1a) and 600 ml of dimethylacetamide, and combined with 61.1 ml (550 mmol) of bromoacetic acid ethyl ester and 410 ml (900 meq) of liquid anion exchanger "Amberlite" LA 2. The mixture is heated for 24 hours to 60° C, the solvent is evaporated after cooling to 25° C, and the oily residue is stirred together with 2 l of n-pentane, thus obtaining a colorless precipitate which, after filtration, is washed repeatedly with n-pentane and then dried under vacuum at 40° C, yielding 38.9 g of a crude product which is recrystallized from 400 ml of ethyl acetate. The product is 31 g (55% of theory) of a colorless crystallized compound, mp 116-117° C.
Analysis:
C 48.49 H 7.45 N 7.39 Br 14.06 (calculated)
C 48.80 H 7.51 N 7.20 Br 13.81 (found)
The desired tetraester (hydrobromide) can be prepared in the same way with the use of lithium carbonate or sodium carbonate as the auxiliary base.

Example 3

2,6-Bis[N-carboxymethyl-N-(2,3-dihydroxy-N-methylpropylcarbamoylmethyl)aminomethyl]-l-piperidineacetic Acid

4.0 g (l0 mmol) of 2,6-bis(2,6-dioxomorpholinomethyl)-l-piperidineacetic acid, dissolved in 50 ml of dry N-methylpyrrolidone, is combined with 2.l g (20 mmol) of N-methylaminopropane-2,3-diol. The solution is heated for 5 hours to 60° C, agitated for l6 hours at room temperature, and the solvent is removed under vacuum. The residue is stirred into l00 ml of diethyl ether, thus obtaining a colorless precipitate which is filtered off and dried under vacuum, yielding 4.6 g (76% of theory) of a white powder, mp l04-l08° C.

Analysis:
C 49.42 H 7.47 N ll.53 (calculated)
C 49.27 H 7.l9 N ll.67 (found)

The starting material is produced as follows:

(a) 2,6-Bis(2,6-dioxomorpholinomethyl)-l-piperidineacetic Acid

4.3 g (l0 mmol) of 2,6-bis[N,N-bis(carboxymethyl)aminomethyl]-l-piperidineacetic acid (prepared according to Example l) is dissolved under heating in 80 ml of dimethylformamide, and the solution is then cooled to 5° C. Under agitation and cooling, 4.2 g (20 mmol) of dicyclohexylcarbodiimide, solid, is added thereto. While continuing cooling over a period of l2 hours, the thus-produced dicyclohexylurea is separated as a white, bulky precipitate. The mixture is filtered, and the filtrate is concentrated under vacuum at maximally 50° C, thus obtaining a light-yellow oil from which the desired anhydride can be precipitated by stirring with diverse organic solvents, preferably with organic ethers such as diethyl ether. A white powder is obtained which, however, hydrolyzes in the air. Therefore, the desired anhydride is identified by IR spectroscopy in the dimethylformamide solution (anhydride bands at l820 and l780 $cm^{-1}$ and COOH at l640 $cm^{-1}$); the solution is concentrated under vacuum, and the residue is taken up, for reaction with the hydroxyalkylamines, in the desired solvent, preferably in N-methyl-2-pyrrolidone.

Example 4

2,6-Bis[N-carboxymethyl-N-[bis(2-hydroxyethyl)carbamoylmethyl]aminomethyl]-l-piperidineacetic Acid

9.9 g (25 mmol) of 2,6-bis(2,6-dioxomorpholinomethyl)-l-piperidineacetic acid (prepared according to Example 3a), dissolved in l50 ml of dry dimethylformamide, is combined with 5.25 g (50 mmol) of diethanolamine, and the solution is heated to 50° C for 5 hours. The solution is then stirred for l6 hours at room temperature and the solvent is removed under vacuum. The residue is stirred with l50 ml of diisopropyl ether, thus obtaining a colorless precipitate. After filtration and drying under vacuum, l2.2 g (82% of theory) of a colorless powder is obtained, mp l4l-l46° C.

Analysis:
C 49.42 H 7.46 N ll.53 (calculated)
C 49.70 H 7.32 N ll.4l (found)

Analogously there are obtained:
with ethylamine:
2,6-bis[N-carboxymethyl-N-[bis(ethyl)carbamoylmethyl]aminomethyl]-l-piperidineacetic acid;
with morpholine:
2,6-bis[N-carboxymethyl-N-[bis(morpholinyl)carbamoylmethyl]aminomethyl]-l-piperidineacetic acid.

Example 5

2,6-Bis[N-carboxymethyl-N-(2,3,4-trihydroxybutylcarbamoylmethyl)aminomethyl]-l-piperidineacetic Acid

4.0 g (l0 mmol) of 2,6-bis(2,6-dioxomorpholinomethyl)-l-piperidineacetic acid (prepared according to Example 3), dissolved in 50 ml of dry N-methylpyrrolidone, is combined with 3.2 g (20 mmol) of 2-amino-l-(2,2-dimethyl-l,3-dioxolan-4-yl)ethanol and heated for 4 hours to 60° C. Then the mxiture is stirrred at room temperature for l6 hours, the solvent is removed under vacuum, and the residue is stirred into l50 ml of diisopropyl ether. The resultant precipitate is filtered and taken up in l50 ml of water, acidified with concentrated hydrochloric acid to pH l, stirred overnight at room teperature, and concentrated under vacuum. The residue is extracted repeatedly by stirring in diisopropyl ether, filtered, and dried under vacuum, thus obtaining 4.3 g (68% of theory) of a colorless powder, mp ll2-ll5° C.

Analysis:
C 46.94 H 7.09 N l0.95 (calculated)
C 46.7l H 7.l8 N ll.l0 (found)

Example 6

Gadolinium(III) Complex of 2,6-bis[N,N-bis(carboxymethyl)aminomethyl]-l-piperidineacetic Acid

43.3 g (l00 mmol) of 2,6-bis[N,N-bis(carboxymethyl)aminomethyl]-l-piperidineacetic acid is suspended in 300 ml of water and combined with l8.l3 g (50 mmol) of gadolinium oxide. The mixture is heated for 3 hours to

l00° C, filtered, and the filtrate concentrated under vacuum. The residue is dried under vacuum at 60° C, thus obtaining 58.l g (99% of theory) of a white powder, the melting point of which lies above 320° C.
Analysis:
C 34.75 H 4.l2 N 7.l5 Gd 26.76 (calculated)
C 34.62 H 4.27 N 7.29 Gd 26.32 (found)

Analogously, by reacting the complexing agent with $^{153}$gadolinium chloride, the corresponding radioactive complex is obtained.

In an analogous way, by reacting the complexing agent with $^{111}$indium chloride (5 mCi/0.l ml), the corresponding $^{111}$indium complex is obtained.

Example 7

Mono-N-methylglucamine Salt of the Gadolinium(III) Complex of 2,6-Bis[N,N-bis(carboxymethyl)aminomethyl]-l-piperidineacetic Acid

8.67 g (20 mmol) of 2,6-bis[N,N-bis(carboxymethyl)aminomethyl]-l-piperidineacetic acid is suspended in 50 ml of water and combined with 3.62 g (l0 mmol) of gadolinium oxide. The mixture is heated to l00° C and, after l5 minutes, 3.90 g (20 mmol) of N-methylglucamine is added. The mixture is maintained for another 2 hours at l00° C, filtered to eliminate minor turbidity, and the filtrate is concentrated under vacuum. The residue is dried under vacuum at 60° C, thus obtaining l5.25 g (97% of theory) of a white powder, mp l85-l90° C.
Analysis:
C 36.82 H 5.28 N 7.l6 Gd 20.09 (calculated)
C 37.0l H 5.02 N 7.0l Gd l9.85 (found)

Example 8

N-Methylglucamine Salt of the Gadolinium(III) Complex of 2,6-Bis[N,N-bis(carboxymethyl)aminomethyl]piperidine

A suspension of l.78 g (4.7 mmol) of 2,6-bis[N,N-bis(carboxymethyl)aminomethyl]piperidine and 0.85 g (2.35 mmol) of gadolinium oxide in 5 ml of water is heated for 2 hours to 80° C, during which step the pH rose to 7.5 by continuous addition of 0.92 g (4.7 mmol) of D-(-)-N-methylglucamine. Subsequently, the mixture was suctioned off over a G4 porous glass filter, and the solution was evaporated on a rotary evaporator under a water jet aspirator vacuum. The residue was dried at 50° C and under 0.l torr, thus obtaining 3.06 g (89% of theory) of a white powder, mp 242-244° C.
Analysis:
C 36.46 H 5.63 N 7.73 Gd 2l.70 (calculated)
C 36.04 H 5.49 N 7.62 Gd 20.93 (found)

Example 9

Gadolinium Complex of 2,6-Bis[N,N-carboxymethyl-N-(2,3-dihydroxy-N-methylpropylcarbamoylmethyl)aminomethyl]-l-piperidineacetic Acid

l5.2 g (25 mmol) of 2,6-bis[N-carboxymethyl-N-(2,3-dihydroxy-N-methylpropylcarbamoylmethyl)aminomethyl]-l-piperidineacetic acid is dissolved in l00 ml of water and combined with 8.4 g (25 mmol) of anhydrous gadolinium acetate. The mixture is stirred for 3 hours at room temperature, and the solution is first poured over an anion exchanger "Amberlite" IRA 4l0 and then the aqueous eluate is poured over a cation exchanger "Amberlite" IRC 50. The mixture is again elutaed with water, and the eluate is concentrated under vacuum, thus obtaining after drying of the residue l3.6 g (7l% of theory) of a colorless powder, mp 2l8-220° C.
Analysis:
C 39.4l H 5.56 N 9.l9 Gd 20.64 (calculated)
C 39.l7 H 5.70 N 9.27 Gd 20.50 (found)

Example l0

Gadolinium Complex of 2,6-Bis[N-carboxymethyl-N-[bis(2-hydroxyethyl)carbamoyl]aminomethyl]-l-piperidineacetic Acid

7.6 g (l2.5 mmol) of 2,6-bis[N-carboxymethyl-N-[bis(2-hydroxyethyl)carbamoylmethyl]aminomethyl]-l-piperidineacetic acid is dissolved in 70 ml of water and combined with 4.2 g (l2.5 mmol) of anhydrous gadolinium acetate. After 4 hours at room temperature under agitation, the solution is poured on an anion exchanger "Amberlite" IRA 4l0. The mixture is eluted with water and the eluate poured on a cation exchanger "Amberlite" IRC 50. The aqueous eluate is concentrated under vacuum and the residue is dried, thus obtaining 6.9 g (73% of theory) of a colorless powder, mp 2l2-2l6° C.
Analysis:
C 39.4l H 5.56 N 9.l9 Gd 20.64 (calculated)
C 39.26 H 5.40 N 9.0l Gd 20.87 (found)

Analogously there are obtained:
gadolinium complex of 2,6-bis[N-carboxy-N-[bis(ethyl)carbamoylmethyl]aminomethyl]-l-piperidineacetic acid; white powder, mp 230-234° C
Analysis:
C 39.30 H 5.34 N l0.9l Gd 24.50 (calculated)
C 39.40 H 5.44 N l0.80 Gd 24.25 (found)
gadolinium complex of 2,6-bis[N-carboxymethyl-N-[bis(morpholinyl)carbamoylmethyl]aminomethyl]-l-piperidineacetic acid;
white powder, mp 238-242° C
Analysis:
C 40.38 H 5.37 N 9.8l Gd 22.03 (calculated)
C 40.50 H 5.5l N 9.7l Gd 22.20 (found)

Example ll

Gadolinium Complex of
2,6-Bis[N-carboxymethyl-N-(2,3,4-trihydroxybutylcarbamoylmethyl)aminomethyl]-l-piperidineacetic Acid

l8.0 g (25 mmol) of 2,6-bis[N-carboxymethyl-N-(2,3,4-trihydroxybutylcarbamoylmethyl)aminomethyl]-l-piperidineacetic acid is dissolved in l50 ml of water and combined with 8.4 g (25 mmol) of anhydrous gadolinium acetate. The mixture is stirred for 4 hours at room temperature; the solution is first poured on an anion exchanger and then on a cation exchanger, as disclosed in Examples 4 and 5, and the eluate is concentrated under vacuum, thus obtaining, after drying, l5.3 g (70% of theory) of a colorless powder, mp 240-242° C.
Analysis:
C 37.82 H 5.33 N 8.82 Gd l9.8l (calculated)
C 37.69 H 5.56 N 8.96 Gd l9.62 (found)

Example l2

Preparation of a Solution of the Di-N-methylglucamine Salt of the Gadolinium(III) Complex of
2,6-Bis[N,N-bis(carboxymethyl)aminomethyl]-l-piperidineacetic Acid

58.76 g (0.l mol) of the gadolinium(III) complex of 2,6-bis[N,N-bis(carboxymethyl)aminomethyl]-l-piperidineacetic acid obtained in Example l is suspended in 40 ml of water p.i. After addition of 0.l8 g of tromethamine hydrochloride and 39.l g (0.2 mol) of N-methylglucamine, the mixture is made into a neutral solution, the solution is filled up to l00 ml with water p.i., dispensed into ampoules, and heat-sterilized.

Example l3

Preparation of a Solution of the Dilysine Salt of the Gadolinium(III) Complex of
2,6-Bis[N,N-bis(carboxymethyl)aminomethyl]-l-piperidineacetic Acid

293.8 g (0.5 mol) of the gadolinium(III) complex of 2,6-bis[N,N-bis(carboxymethyl)aminomethyl]-l-piperidineacetic acid is suspended in 500 ml of water p.i. The suspension is combined with 292.4 g (l mol) of lysine, agitated for several hours with slight heating, and then filled up with water p.i. to give l,000 ml. The solution is dispensed into bottles and heat-sterilized.

Example l4

Preparation of a Solution of the Sodium Salt of the Gadolinium(III) Complex of
2,6-Bis[N,N-bis(carboxymethyl)aminomethyl]-l-piperidineacetic Acid

587.64 g (l mol) of the gadolinium(III) complex of 2,6-bis[N,N-bis(carboxymethyl)aminomethyl]-l-piperidineacetic acid is suspended in 500 ml of water p.i. and made into a neutral solution by adding, in portions, 40 g (l mol) of sodium hydroxide. After adding l.5 g of tromethamine, the solution is filled up to give l,000 ml with water p.i., dispensed into bottles, and heat-sterilized.

Example l5

Preparation of a Solution of the Gadolinium(III) Complex of the Conjugate of
2,6-Bis[N,N-bis(carboxymethyl)aminomethyl]-l-piperidineacetic Acid with Human Serum Albumin

l0 mg of 2,6-bis(2,6-dioxomorpholinomethyl)-l-piperidineacetic acid, dissolved in 0.l ml of dimethylformamide, is added to 20 ml of a solution of 3 mg of the protein in 0.05-molar sodium bicarbonate buffer (pH 7-8). The mixture is agitated for 30 minutes at room temperature and then dialyzed against a 0.3-molar sodium phosphate buffer. Then 50 mg of gadolinium(III) acetate is added and the mixture is purified by gel chromatography on a "Sephadex" G25 column. The thus-obtained fraction is filtered under sterile conditions and dispensed into multivials. By freeze-drying, a storable dry preparation is obtained, gadolinium content l.8%.

Analogously, using immunoglobulin, a solution of the corresponding complex conjugate is obtained having

a gadolinium content of 1.1%.
In an analogous way, using hydroxyethyl starch, a solution of the corresponding complex conjugate is produced with a gadolinium content of 2.1%.

Example 16

Preparation of Liposomes Carrying Gadolinium(III) 2,6-Bis[N,N-bis(carboxymethyl)aminomethyl]-1-piperidineacetic Acid

According to the procedure described in Proc. Natl. Acad. Sci. U.S.A. 75 : 4194, a lipid mixture is prepared from 75 mol-% Egg-phosphatidylcholine and 25 mol-% cholesterol as a dry substance. Of this substance, 500 mg is dissolved in 30 ml of diethyl ether and combined, in an ultrasonic bath, dropwise with 3 ml of a 0.1-molar solution of the di-N-methylglucamine salt of the gadolinium(III) complex of 2,6-bis[N,N-bis-(carboxymethyl)aminomethyl]-1-piperidineacetic acid in water p.i. After the entire solution has been added, the ultrasonic treatment is continued for 10 minutes, and then the mixture is concentrated in a "Rotavapor" rotary evaporator. The gel-like residue is suspended in 0.125-molar sodium chloride solution and freed at 0° C repeatedly by centrifuging (20,000 g/20 minutes) from not encapsulated contrast media proportions. Subsequently, the thus-obtained liposomes are freeze-dried in a multivial. Administration takes place as a colloidal dispersion in 0.9% by weight sodium chloride solution.

Example 17

Preparation of a Solution of the $^{90}$Yttrium Complex of the Conjugate of 2,6-Bis[N,N-bis(carboxymethyl)aminomethyl]-1-piperidineacetic Acid with Monoclonal Antibody

1 mg of 2,6-bis(2,6-dioxomorpholinomethyl)-1-piperidineacetic acid, dissolved in 0.01 ml of dimethylformamide, is added to 1 ml of a solution of 0.15 mg of monoclonal antibody (having specificity against melanoma antigen) in 0.05-molar sodium bicarbonate buffer (pH 7-8). The mixture is stirred for 30 minutes at room temperature and dialyzed against a 0.3-molar sodium phosphate buffer. Then 250 µl of a $^{90}$Y solution in acetate buffer (pH 6; prepared in accordance with Int. J. Appl. Radiat. Isot. 36 : 803 [1985]) is added thereto and the mixture is incubated for 15 minutes at room temperature. The solution is passed over a combined "Sephadex" G25 column, and the radioactive protein fraction is filtered under sterile conditions and dispensed into multivials. By lyophilization, a storable dry preparation is obtained.

Example 18

Mono-N-methylglucamine Salt of the Manganese(II) Complex of 2,6-Bis[N,N-bis(carboxymethyl)aminomethyl]-1-piperidineacetic Acid

8.67 g (20 mmol) of 2,6-bis[N,N-bis(carboxymethyl)aminomethyl]-1-piperidineacetic acid is suspended in 30 ml of water and combined with 1.4 g (20 mmol) of manganese(II) oxide MnO. The mixture is heated to 100° C and, after 3 hours, 3.90 g (20 mmol) of N-methylglucamine is added thereto. The mixture is maintained at 100° C for another 12 hours, then the solution is concentrated to dryness under vacuum, thus obtaining in a quantitative yield a pink powder, mp 130-135° C.

Analysis:
C 42.29 H 6.21 N 8.22 Mn 8.06 (calculated)
C 42.40 H 6.10 N 8.31 Mn 8.15 (found)

Example 19

Mono-N-methylglucamine Salt of the Dysprosium(III) Complex of 2,6-Bis[N,N-bis(carboxymethyl)aminomethyl]-1-piperidineacetic Acid

8.67 g (20 mmol) of 2,6-bis[N,N-bis(carboxymethyl)aminomethyl]-1-piperidineacetic acid is suspended in 30 ml of water and combined with 3.73 g (10 mmol) of dysprosium oxide. The mixture is heated to 100° C and, after 15 minutes, 3.90 g (20 mmol) of N-methylglucamine is added. The mixture is maintained for 5 hours at 100° C and the solution is concentrated to dryness under vacuum, thus obtaining 15.76 g (100% of theory) of a white powder, mp 182-185° C.

Analysis:
C 36.58 H 5.24 N 7.11 Dy 20.62 (calculated)
C 36.44 H 5.40 N 7.21 Dy 21.50 (found)

Example 20

Gadolinium(III) Complex of 2,6-Bis[(N-carboxymethyl-N-isopropoxycarbonylmethyl)aminomethyl]-1-piperidineacetic Acid

4.0 g (10 mmol) of 2,6-bis(2,6-dioxomorpholinomethyl)-1-piperidineacetic acid (prepared according to Example 3) is heated to reflux for five hours with 20 ml of isopropanol. The mixture is then concentrated to dryness under vacuum, and the residue is heated in 100 ml of water with 1.8 g (5 mmol) of gadolinium oxide until

a clear solution is obtained. Subsequently the solution is freeze-dried. The product is obtained as a white foam with a melting point lying above 300° C.

Analysis:

C 41.18 H 5.26 N 6.26 Gd 41.18 (calculated)

C 41.22 H 5.40 N 6.12 Gd 40.95 (found)

## Claims

1. Physiologically compatible complex compounds of general Formula I

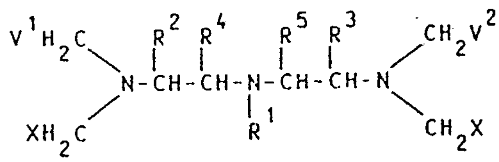

(I)

wherein

X means the residues -COOY and $PO_3HY$ with Y meaning a hydrogen atom, a metal ion equivalent and/or a physiologically acceptable cation of an inorganic or organic base or amino acid,

$V^1$ is the residue X, a

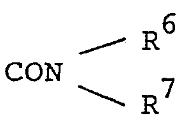

or $COOR^{12}$ group wherein

$R^6$ stands for a saturated, unsaturated, straight- or branched-chain or cyclic hydrocarbon residue of up to 16 carbon atoms optionally substituted by 1-5 hydroxy groups or an aryl or aralkyl group optionally substituted by one or several $C_1$-$C_6$-dialkylamino or one or several $C_1$-$C_6$-alkoxy groups,

$R^7$ stands for a hydrogen atom, for a saturated, unsaturated, straight- or branched-chain or cyclic hydrocarbon residue of up to 16 carbon atoms optionally substituted by 1-5 hydroxy groups, or $R^6$ and $R^7$ jointly stand for a saturated or unsaturated 5- or 6-membered ring optionally substituted by one or several $C_1$-$C_5$-alkyl, $C_1$-$C_5$-hydroxyalkyl, an optionally hydroxylated or $C_1$-$C_5$-alkoxylated $C_2$-$C_6$-acyl, hydroxy, carbamoyl, carbamoyl-substituted $C_1$-$C_6$-alkyl, carbamoyl substituted at nitrogen by one or two $C_1$-$C_6$-alkyl residue(s) -- which also can form a ring optionally containing an oxygen atom -- or a $C_1$-$C_5$-acylamino or alkylacylamino residue, this 5- or 6-membered ring optionally containing a further nitrogen, oxygen, sulfur atom, or a carbonyl group, and

$R^{12}$ stands for a saturated, unsaturated, straight- or branched-chain or cyclic hydrocarbon residue of up to 16 carbon atoms, or an aryl or aralkyl group,

$V^2$ means the residue X,

a

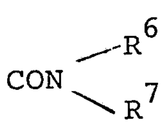 ,

$COOR^{12}$ or COB group wherein B is a macromolecule residue,

$R^1$ is a hydrogen atom or a $CH_2X$ group,

$R^2$ and $R^3$ mean a $(CH_2)_m$ group with m meaning 0 or 1 if $R^4$ and $R^5$ are simultaneously a hydrogen atom each, $R^4$ and $R^5$ are a

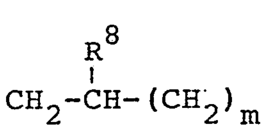

group with m meaning 0 or l,
$R^8$ meaning a hydrogen atom or $R^9$,
wherein $R^9$ stands for a straight-chain or branched, saturated or unsaturated $C_1$-$C_{20}$-alkylene group which exhibits at the end either a functional group or, bound via the latter, a macromolecule B and contains optionally imino, phenylenoxy, phenylenimino, amide, ester group(s), oxygen, sulfur and/or nitrogen atom(s) and is optionally substituted by hydroxy, mercapto, imino and/or amino group(s), if simultaneously $R^2$ and $R^3$ each stand for a hydrogen atom,
with the proviso that, if $V^2$ stands for a COB group, $R^8$ means a hydrogen atom and that, if X stands for the group $PO_3HY$, $V^1$ and $V^2$ likewise mean $PO_3HY$.

2. Compounds according to claim l, characterized in that Y stands in each case for a hydrogen atom.

3. 2,6-Bis[N,N-bis(carboxymethyl)aminomethyl]-l-piperidineacetic acid;
2,6-bis[N,N-bis(carboxymethyl)aminomethyl]piperidine;
2,6-bis[N,N-carboxymethyl-N-(2,3-dihydroxy-N-methylpropylcarbamoylmethyl)aminomethyl]-l-piperidineacetic acid;
2,6-bis[N-carboxymethyl-N-[bis(2-hydroxyethyl)carbamoylmethyl]aminomethyl]-l-piperidineacetic acid;
2,6-bis[N-carboxymethyl-N-(2,3,4-trihydroxybutylcarbamoylmethyl)aminomethyl]-l-piperidineacetic acid;
2,6-bis[N-carboxymethyl-N-[bis(ethyl)carbamoylmethyl]aminomethyl]-l-piperidineacetic acid;
2,6-bis[N-carboxymethyl-N-[bis(morpholinyl]carbamoylmethyl]aminomethyl]-l-piperidineacetic acid.

4. Physiologically compatible complex compounds according to claim l, characterized in that at least two of the substituents X are metal ion equivalents of at least one element of the atomic numbers 2l-29, 42, 44 or 57-70.

5. Physiologically compatible complex compounds according to claim l, characterized in that at least two of the substituents X are metal ion equivalents of at least one radionuclide of an element of the atomic numbers 27, 29, 3l, 32, 38, 39, 43, 49, 64, 70 or 77.

6. Compounds according to claim l, characterized in that $V^1$ and/or $V^2$ stand in each case for a

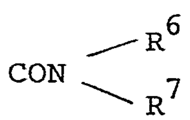

group.

7. Compounds according to claim l, characterized in that $V^1$ and/or $V^2$ stand in each case for the residue X.

8. Compounds according to claim l, characterized in that $V^1$ and/or $V^2$ stand in each case for a $COOR^{12}$ group.

9. Compounds according to claim l, characterized in that $V^2$ stands for a COB group.

l0. Compounds according to claim l, characterized in that $R^1$ stands for a hydrogen atom.

ll. Compounds according to claim l, characterized in that $R^1$ stands for the residue X.

l2. Compounds according to claim l, characterized in that $R^2$ and $R^3$ are a $(CH_2)_m$ group and at the same time $R^4$ and $R^5$ each stand for a hydrogen atom.

l3. Compounds according to claim l, characterized in that $R^4$ and $R^5$ represent a

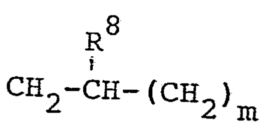

group and at the same time $R^2$ and $R^3$ each stand for a hydrogen atom.

l4. Gadolinium(III) complex of 2,6-bis[N,N-bis(carboxymethyl)aminomethyl]-l-piperidineacetic acid;
mono-N-methylglucamine salt of the gadolinium(III) complex of 2,6-bis[N,N-bis(carboxymethyl)aminomethyl]-l-piperidineacetic acid;
N-methylglucamine salt of the gadolinium(III) complex of 2,6-bis[N,N-bis(carboxymethyl)aminomethyl-]piperidine;
gadolinium complex of 2,6-bis[N,N-carboxymethyl-N-(2,3-dihydroxy-N-methylpropylcarbamoylmethyl)aminomethyl]-l-piperidineacetic acid;
gadolinium complex of 2,6-bis[N-carboxymethyl-N-[bis(2-hydroxyethyl)carbamoyl]aminomethyl]-l-piperidineacetic acid;
gadolinium complex of 2,6-bis[N-carboxymethyl-N-(2,3,4-trihydroxybutylcarbamoylmethyl)aminomethyl]-l-piperidineacetic acid;
gadolinium complex of 2,6-bis[N-carboxy-N-[bis(ethyl)carbamoylmethyl]aminomethyl]-l-piperidineacetic acid;
gadolinium complex of 2,6-bis[N-carboxymethyl-N-[bis(morpholino)carbamoylmethyl]aminomethyl]-l-piperidineacetic acid;
gadolinium(III) complex of the conjugate of 2,6-bis[N,N-bis(carboxymethyl)aminomethyl]-l-piperidineacetic acid with human serum albumin;
gadolinium(III) complex of the conjugate of 2,6-bis[N,N-bis(carboxymethyl)aminomethyl]-l-piperidineacetic acid with immunoglobulin;
$^{111}$indium complex of 2,6-bis[N,N-bis(carboxymethyl)aminomethyl]-l-piperidineacetic acid;
gadolinium(III) complex of the conjugate of 2,6-bis[N,N-bis(carboxymethyl)aminomethyl]-l-piperidineacetic acid with hydroxyethyl starch;
mono-N-methylglucamine salt of the manganese(II) complex of 2,6-bis[N,N-bis(carboxymethyl)aminomethyl]-l-piperidineacetic acid;
mono-N-methylglucamine salt of the dysprosium(III) complex of 2,6-bis[N,N-bis(carboxymethyl)aminomethyl]-l-piperidineacetic acid;
gadolinium(III) complex of 2,6-bis[(N-carboxymethyl-N-isopropoxycarbonylmethyl)aminomethyl]-l-piperidineacetic acid.

l5. Pharmaceutical agents containing at least one physiologically compatible complex compound according to claims 4, 5 and l4, optionally with the additives customary in galenic pharmacy.

l6. Use of the compounds according to claims 2 and 3 as complexing agents.

l7. Use of at least one physiologically compatible complex compound according to claim 4 for the preparation of media for NMR, X-ray or ultrasonic diagnostics.

l8. Use of at least one physiologically compatible complex compound according to claim 5 for the preparation of media for radiodiagnostics and radiotherapy.

l9. Process for the preparation of compounds of general Formula I according to claim I, characterized in that, in a manner known per se, amines of general Formula II

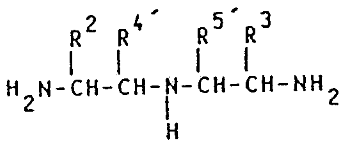

(II),

wherein
$R^2$ and $R^3$ represent a $(CH_2)_m$ group where m means 0 or l if simultaneously $R^{4'}$ and $R^{5'}$ each stand for a hydrogen atom,
$R^{4'}$ and $R^{5'}$ represent a

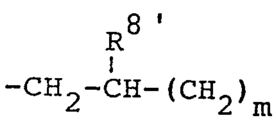

group wherein $R^{8'}$ is a hydrogen atom or $R^{9'}$, wherein $R^{9'}$ stands for a straight-chain, branched, saturated or unsaturated $C_1$-$C_{20}$-alkylene group exhibiting at the end a group that can be converted into the functional group contained in $R^9$ and optionally containing imino, phenylenoxy, phenylenimino, amide ester group(s), oxygen, sulfur and/or nitrogen atom(s) and being optionally substituted by hydroxy, mercapto, imino and/or amino group(s),
are alkylated with

(a) phosphorous acid/formaldehyde or in the presence of a base

(b) a compound $HalCH_2COOR^{10}$ wherein Hal is chlorine, bromine or iodine and $R^{10}$ is a hydrogen atom or an alkyl residue of l-4 carbon atoms,

and subsequently alkyl residues $R^{10}$ that may be present are split off by saponification and, if desired, the thus-obtained acetic-acid-substituted compounds are reacted, by way of compound III, IV or V

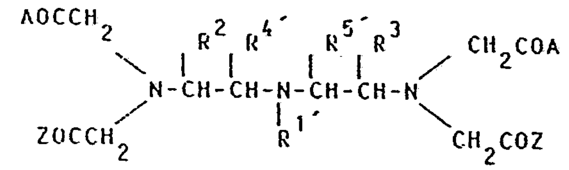 (III),

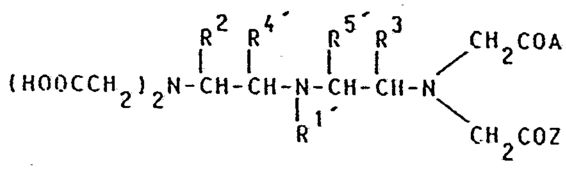 (IV),

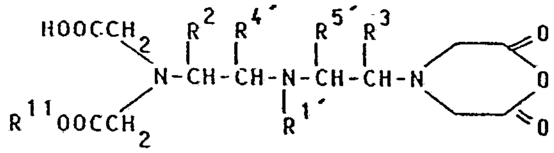 (V),

wherein
$R^{1'}$ is a hydrogen atom or a $CH_2COOH$ group,
$R^{11}$ is a $C_1$-$C_6$-alkyl residue,
A and Z jointly represent an oxygen atom, or A is a hydroxy group and Z is the grouping $OR^{11}$, with an amine of general Formula VI

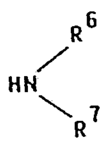 (VI),

and optionally still present ester groups are saponified or, respectively, bisanhydrides of general Formula IIIa

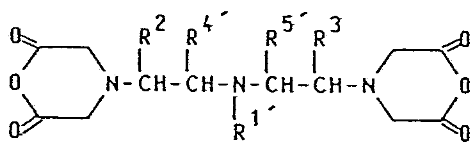 (IIIa)

are reacted with an alcohol $R^{12}OH$,
the thus-obtained acids of general Formula I wherein Y means a hydrogen atom, if desired,

(a) are conventionally reacted with at least one metal oxide or metal salt of an element of atomic numbers 2l-29, 3l, 32, 38, 39, 42-44, 49 or 57-70 or 77 and subsequently, if desired, acidic hydrogen atoms present are substituted by cations of inorganic and/or organic bases or amino acids,
or

(b) are conventionally reacted with at least one metal oxide or metal salt of an element of atomic numbers 2l-29, 3l, 32, 38, 39, 42-44, 49, 57-70 or 77 and subsequently the thus-obtained metal complexes are conventionally bound to a macromolecule by way of the functional group contained in $R^{9'}$ and, respectively, to the CO group contained in $V^2$, and, if desired, acidic hydrogen atoms present are substituted by cations of inorganic and/or organic bases or amino acids,
or

(c) are bound conventionally to a macromolecule by way of the functional group contained in $R^{9'}$ and, respectively, to the CO group contained in $V^2$, and subsequently, in a manner known per se, are reacted with at least one metal oxide or metal salt of an element of atomic numbers 2l-29, 3l, 32, 38, 39, 42-44, 49, 57-70 or 77 and thereafter, if desired, acidic hydrogen atoms present are substituted by cations of inorganic and/or organic bases or amino acids.

20. Process for the preparation of the pharmaceutical agents according to claim l5, characterized in that

the complex compound, dissolved or suspended in water or physiological saline solution, is brought, optionally with the additives customary in galenic pharmacy, into a form suitable for enteral or parenteral administration.